(19) Europäisches Patentamt
European Patent Office
Office européen des brevets

(11) Veröffentlichungsnummer: **0 286 968**
**A2**

(12) # EUROPÄISCHE PATENTANMELDUNG

(21) Anmeldenummer: **88105500.8**

(22) Anmeldetag: **07.04.88**

(51) Int. Cl.4: **C07D 231/12 , A01N 43/56 , //C07D231/08,C07C109/087, C07C109/14**

(30) Priorität: **16.04.87 DE 3712934**

(43) Veröffentlichungstag der Anmeldung: **19.10.88 Patentblatt 88/42**

(84) Benannte Vertragsstaaten: **AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Ooms, Pieter, Dr.**
**Doerperhofstrasse 16**
**D-4150 Krefeld 1(DE)**
Erfinder: **Becker, Benedikt, Dr.**
**Metzausener Strasse 14**
**D-4020 Mettmann(DE)**
Erfinder: **Behrenz, Wolfgang, Dr.**
**Untergründemich 14**
**D-5063 Overath(DE)**
Erfinder: **Homeyer, Bernhard, Dr.**
**Obere Strasse 28**
**D-5090 Leverkusen 3(DE)**

(54) **Substituierte 1-Arylpyrazole.**

(57) Es werden neue substituierte 1-Arylpyrazole der allgemeinen Formel (I)

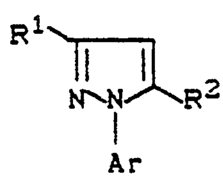

(I)

bereitgestellt,
in welcher
$R^1$ für Halogenalkyl steht,
$R^2$ für Wasserstoff, Alkyl, Cycloalkyl oder Halogenalkyl steht und
Ar für substituiertes Phenyl mit Ausnahme des 4-Nitrophenylrestes, des 2,4-Dinitrophenylrestes und mit Ausnahme von 2,4-Dihalogen-5-alkoxy-substituierten Phenylresten steht.

Die neuen Verbindungen der Formel (I) werden nach an sich bekannten Verfahren hergestellt und sie besitzen eine ausgezeichnete Wirkung als Schädlingsbekämpfungsmittel, insbesondere als Insektizide.

EP 0 286 968 A2

## Substituierte 1-Arylpyrazole

Die Erfindung betrifft neue substituierte 1-Arylpyrazole, mehrere Verfahren zu ihrer Herstellung und ihre Verwendung als Schädlingsbekämpfungsmittel.

Es ist bereits bekannt, daß bestimmte 1-Arylpyrazole wie beispielsweise das 4-Cyano-5-propionamido-1-(2,3,4-trichlorphenyl)-pyrazol oder das 4-Cyano-5-propionamido-1-(2,4,6-trichlorphenyl)-pyrazol herbizide Eigenschaften besitzen (vgl, z. B. DE-OS 32 26 513).

Weiterhin sind bestimmte substituierte 1-Arylpyrazole, wie beispielsweise das 3,5-Bis-(chlormethyl)-1-(2,4-dinitrophenyl)-pyrazol oder das 3-Chlormethyl-1-(2,4-dinitrophenyl)-pyrazol oder das 3-Chlormethyl-1-(4-nitrophenyl)-pyrazol oder das 3-Trifluormethyl-1-(2-Chlor-4-fluor-5-ethoxy-phenyl)-pyrazol oder das 3-Trifluormethyl-5-methyl-1-(2-fluor-4-chlor-5-isopropoxy-phenyl)-pyrazol oder das 3,5-Bis-(trifluormethyl)-1-(2-fluor-4-chlor-5-isopropoxy-phenyl)-pyrazol oder das 3,5-Bis-(trifluormethyl)-1-(2,4-dichlor-5-isopropoxy-phenyl)-pyrazol oder das 3,5-Bis-(trifluormethyl)-1-(2-chlor-4-brom-5-isopropoxyphenyl)-pyrazol oder das 3,5-Bis-(trifluormethyl)-1-(2-brom-4-chlor-5-isopropoxy-phenyl)-pyrazol, bekannt. Die vorgenannten Verbindungen sind aus folgenden Publikationen bekannt: EP 202 169; Khim.-Farm. Zh. 18, 173-175, [1984] bzw. CA 100: 2097098; Ukr. Khim. Zh. (russ. Ed.) 50, 307 -311 [1984] bzw. C.A. 101: 71807 h.

Über eine insektizide Wirksamkeit dieser vorbekannten Verbindungen ist jedoch nichts bekannt geworden.

Es wurden neue substituierte 1-Arylpyrazole der allgemeinen Formel (1),

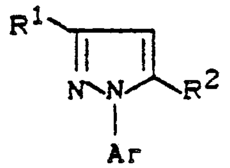

(I)

in welcher
$R^1$ für Halogenalkyl steht,
$R^2$ für Wasserstoff, Alkyl, Cycloalkyl oder Halogenalkyl steht und
Ar für substituiertes Phenyl mit ausnahme des 4-Nitrophenylrestes, des 2,4-Dinitrophenylrestes und mit Ausnahme von 2,4-Dihalogen-5-alkoxy-substituierten Phenylresten steht,
gefunden.

Weiterhin wurde gefunden, daß man die neuen substituierten 1-Arylpyrazole der allgemeinen Formel (I),

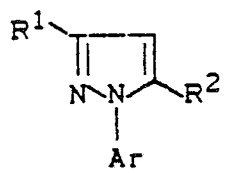

(I)

in welcher
$R^1$ für Halogenalkyl steht,
$R^2$ für Wasserstoff, Alkyl, Cycloalkyl oder Halogenalkyl steht und
Ar für substituiertes Phenyl mit Ausnahme des 4-Nitrophenylrestes, des 2,4-Dinitrophenylrestes und mit Ausnahme von 2,4-Dihalogen-5-alkoxy-substituierten Phenylresten steht,
erhält, wenn man

a) N-Arylhydrazid-halogenide der Formel (II),

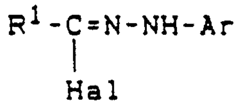

(II)

in welcher
$R^1$ und Ar die oben angegebene Bedeutung haben und
Hal für Halogen steht,
mit Olefinen der Formel (III),

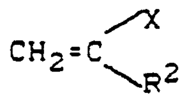

(III)

in welcher
$R^2$ die oben angegebene Bedeutung hat und
X für Alkoxy, Alkylthio, Dialkylamino, Morpholino, Halogen oder Trialkylsilyloxy steht,
gegenbenenfalls in Gegenwart eines Verdünnungsmittels und gegebenenfalls in Gegenwart eines Säurebindemittels umsetzt oder wenn man

(b) Pyrazoline der Formel (IV),

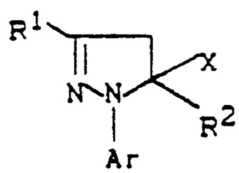

(IV)

in welcher
$R^1$, $R^2$ und Ar die oben angegebene Bedeutung haben und
X für Alkoxy, Alkylthio, Dialkylamino, Morpho lino, Halogen oder Trialkylsilyloxy steht,
mit Säuren gegebenenfalls in Gegenwart eines Verdünnungsmittels umsetzt oder wenn man

(c) 1,3-Diketone der Formel (v),

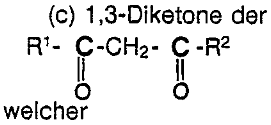

(V)

in welcher
$R^1$ und $R^2$ die oben angegebene Bedeutung haben,
oder Derivate dieser 1,3-Diketone der Formel (Va)

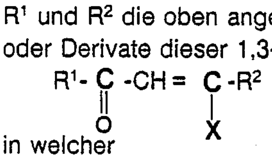

(Va)

in welcher
$R^1$ und $R^2$ die oben angegebene Bedeutung haben und
X für Alkoxy, Alkylthio, Dialkylamino, Morpholino, Halogen oder Trialkylsilyloxy steht,
mit Arylhydrazinen der Formel (VI),

$Ar\text{-}NH\text{-}Nh_2$ (VI)

in welcher
Ar die oben angegebene Bedeutung hat,
gegenbenenfalls in Gegenwart eines Verdünnungsmittels umsetzt oder wenn man

(d) Pyrazolyl-4-carbonsäuren der Formel (VII),

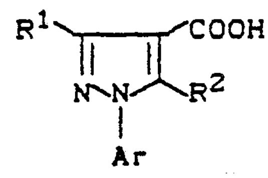

(VII)

in welcher

$R^1$, $R^2$ und Ar die oben angegebene Bedeutung haben,

gegebenenfalls in Gegenwart eines Verdünnungsmittels und gegebenenfalls in Gegenwart eines Reaktionshilfsmittels decarboxyliert.

Schließlich wurde gefunden, daß die neuen substituierten 1-Arylpyrazole der allgemeinen Formel (I) eine gute Wirkung gegen Schädlinge, insbesondere eine insektizide Wirkung besitzen.

Überraschenderweise zeigen die erfindungsgemäßen substituierten 1-Arylpyrazole der allgemeinen Formel (I) eine bessere insektizide Wirksamkeit als die aus dem Stand der Technik bekannten 1-Arylpyrazole, wie beispielsweise das 4-Cyano-5-propionamido-1-(2,3,4-trichlorphenyl)-pyrazol oder das 4-Cyano-5-propionamido-1-(2,4,6-trichlorphenyl)-pyrazol, welches chemisch naheliegende Verbindungen sind.

Die erfindungsgemäßen substituierten 1-Arylpyrazole sind durch die Formel (I) allgemein definiert. Bevorzugt sind Verbindungen der Formel (I), bei welchen

$R^1$ für geradkettiges oder verzweigtes Halogenalkyl mit 1 bis 4 Kohlenstoffatomen und 1 bis 9 gleichen oder verschiedenen Halogenatomen steht,

$R^2$ für Wasserstoff, für jeweils geradkettiges oder verzweigtes Alkyl oder Halogenalkyl mit jeweils 1 bis 4 Kohlenstoffatomen und im Fall des Halogenalkyl mit 1 bis 9 gleichen oder verschiedenen Halogenatomen oder für Cycloalkyl mit 3 bis 7 Kohlenstoffatomen steht und

Ar für einfach oder mehrfach gleich oder verschieden substituiertes Phenyl mit Ausnahme des 4-Nitrophenylrestes, des 2,4-Dinitrophenylrestes und mit Ausnahme von 2,4-Dihalogen-5-alkoxy-substituierten Phenylresten steht, wobei als Substituenten in Frage kommen: Halogen, Cyano, Nitro, sowie jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl oder Alkoxycarbonyl mit jeweils 1 bis 4 Kohlenstoffatomen in den ein zelnen Alkylteilen und gegebenenfalls 1 bis 9 gleichen oder verschiedenen Halogenatomen.

Besonders bevorzugt sind Verbindungen der Formel (I), bei welchen

$R^1$ für Trifluormethyl, Dichlorfluormethyl, Difluorchlormethyl, Difluormethyl, Trifluorethyl, Trifluorchlorethyl, Trifluordichlorethyl, Difluordichlorethyl, Tetrafluorethyl, Pentafluorethyl, Heptafluorpropyl oder Nonafluorbutyl steht,

$R^2$ für Wasserstoff, Methyl, Ethyl, n-oder i-Propyl, n-, i-, s-oder t-Butyl, Trifluormethyl, Dichlorfluormethyl, Difluorchlormethyl, Difluormethyl, Trifluorethyl, Tetrafluorethyl, Pentafluorethyl, Trifluorchlorethyl, Trifluordichlorethyl, Difluordichlorethyl, Heptafluorpopyl, Nonafluorbutyl, für Cyclopropyl, Cyclobutyl, Cyclopentyl, oder Cyclohexyl steht und

Ar für ein-bis fünffach, gleich oder verschieden substituiertes Phenyl mit Ausnahme des 4-Nitrophenylrestes, des 2,4-Dinitrophenylrestes und mit Ausnahme von 2,4-Dihalogen-5-alkoxy-substituierten Phenylresten steht, wobei als Substituenten in Frage kommen: Fluor, Chlor, Brom, Iod, Cyano, Nitro, Methyl, Ethyl, n-oder i-Propyl, n-, i-, s-oder t-Butyl, Methoxy, Ethoxy, Methylthio, Methylsulfinyl, Methylsulfonyl, Methoxycarbonyl, Ethoxycarbonyl, Trifluormethyl, Dichlorfluormethyl, Difluorchlor methyl, Difluormethyl, Pentafluorethyl, Tetrafluorethyl, Trifluorchlorethyl, Trifluorethyl, Difluorchlorethyl, Trifluordichlorethyl oder ein Rest -Y-$R^3$, wobei Y für Sauerstoff, Schwefel, Sulfinyl oder Sulfonyl steht und $R^3$ für Trifluormethyl, Dichlorfluormethyl, Difluorchlormethyl, Difluormethyl, Pentafluorethyl, Tetrafluorethyl, Trifluorchlorethyl, Trifluorethyl, Difluorchlorethyl oder Trifluordichlorethyl steht.

Im einzelnen seien außer den bei den Herstellungsbeispielen genannten Verbindungen die folgenden substituierten 1-Arylpyrazole der allgemeinen Formel (I) genannt:

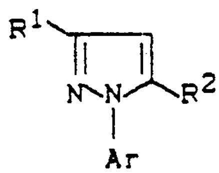 (I)

| $R^1$ | $R^2$ | Ar |
|---|---|---|
| $CF_3$ | $CH_3$ | 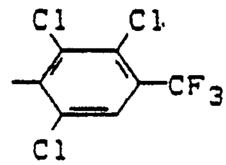 |
| $CF_3$ | $CH_3$ | 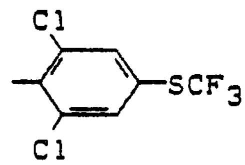 |
| $CF_3$ | $CH_3$ | 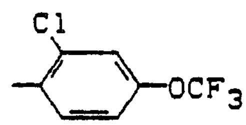 |

| $R^1$ | $R^2$ | Ar |
|---|---|---|
| $CF_3$ | $CH_3$ | 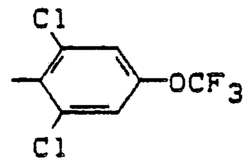 |
| $CF_3$ | $CH_3$ | 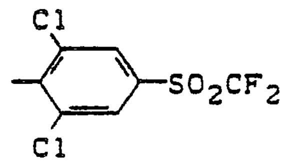 |
| $CF_3$ | $CH_3$ | 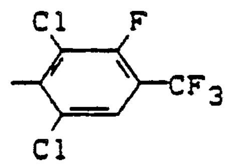 |
| $CF_3$ | $CF_3$ | 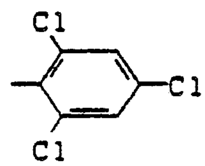 |
| $CF_3$ | $CF_3$ | 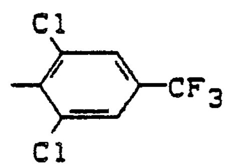 |
| $CF_3$ | $CF_3$ | 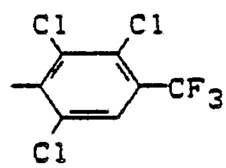 |
| $CF_3$ | $CF_3$ | 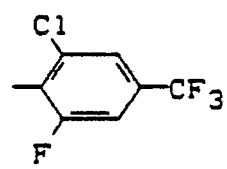 |
| $CF_3$ | $CF_3$ | 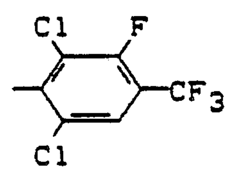 |

| $R^1$ | $R^2$ | Ar |
|---|---|---|
| $CF_3$ | $CF_3$ | 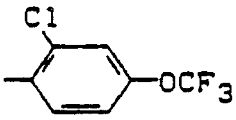 |
| $CF_3$ | $CF_3$ | 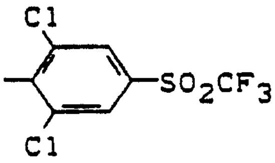 |
| $CF_3$ | $CF_3$ | 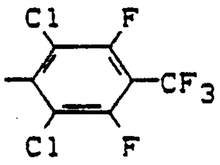 |
| $CF_3$ | $CF_3$ | 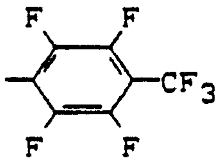 |
| $CF_3$ | $CF_3$ | 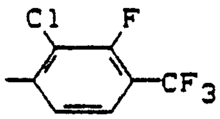 |
| $CF_3$ | $CF_3$ | 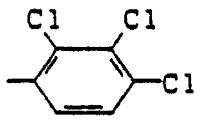 |
| $CF_3$ | $CH_3$ | 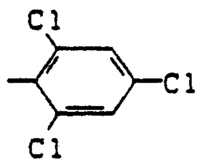 |
| $CF_3$ | $CH_3$ | 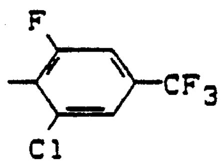 |

| $R^1$ | $R^2$ | Ar |
|---|---|---|
| $CF_3$ | $CH_3$ | 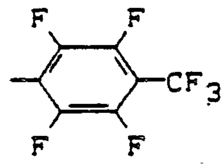 |
| $CF_3$ | H | 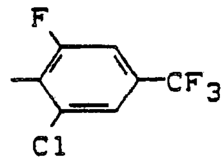 |
| $CF_3$ | H | 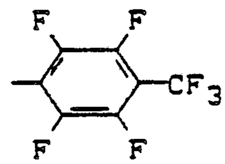 |
| $C_2F_5$ | H | 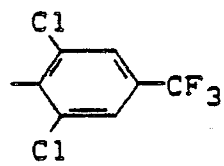 |
| $C_3F_7$ | H | 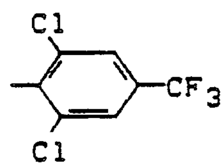 |
| $C_4F_9$ | H | 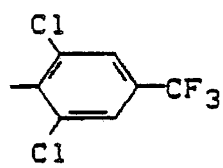 |
| $C_2F_5$ | H | 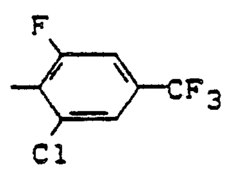 |
| $C_3F_7$ | H | 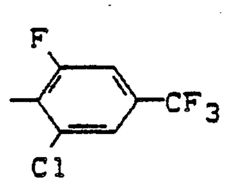 |

| $R^1$ | $R^2$ | Ar |
|---|---|---|
| $C_4F_9$ | H | 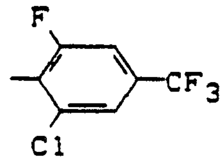 |
| $C_2F_5$ | H | 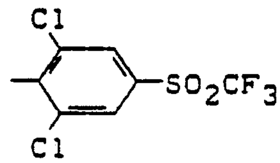 |
| $C_3F_7$ | H | 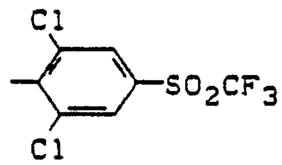 |
| $C_4F_9$ | H | 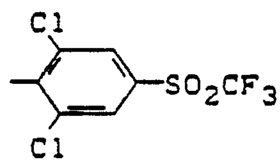 |
| $C_2F_5$ | H | 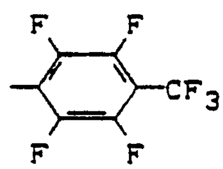 |
| $C_3F_7$ | H | 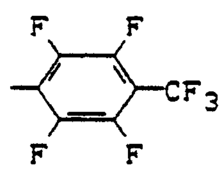 |
| $C_4F_9$ | H | 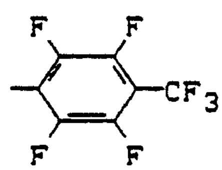 |
| $CF_3$ | H | 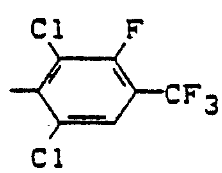 |

| $R^1$ | $R^2$ | Ar |
|---|---|---|
| $C_2F_5$ | H | 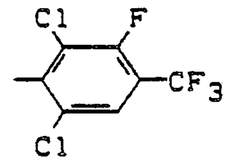 |
| $C_3F_7$ | H | 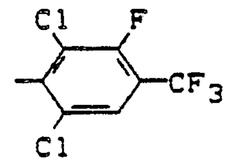 |
| $C_4F_9$ | H | 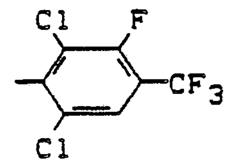 |

Verwendet man beispielsweise N-(2,6-Dichlor-4-trifluormethylphenyl)-trifluoracethydrazid-bromid und 2-Propenylmethylether als Ausgangsstoffe, so läßt sich der Reaktionsablauf des erfindungsgemäßen Verfahrens (a) durch das folgende Formelschema darstellen

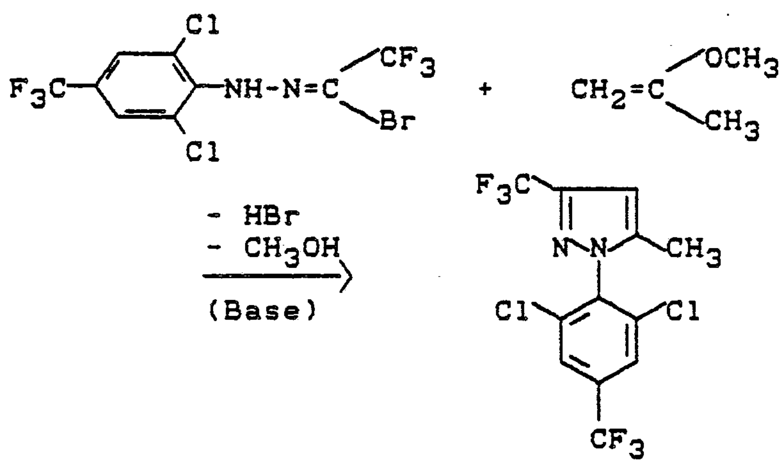


Verwendet man beispielsweise 5-Butoxy-3-trifluormethyl-1-(2,6-dichlor-4-trifluormethylphenyl)-$\Delta^2$-pyrazolin als Ausgangsverbindung, so läßt sich der Reaktionsablauf des erfindungsgemäßen Verfahrens (b) durch das folgende Formelschema darstellen:

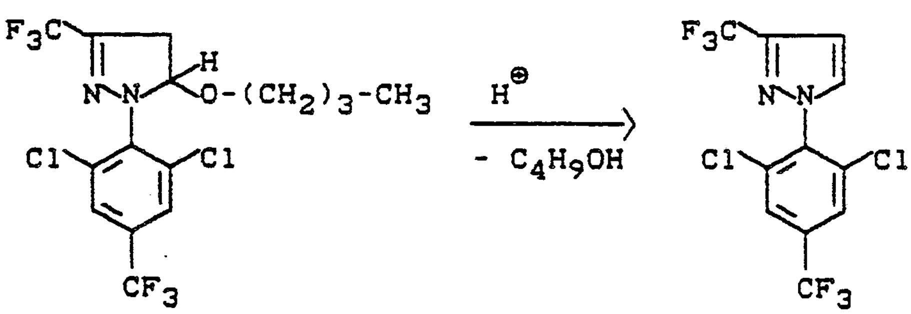

Verwendet man beispielsweise 1,1,1-Trifluorpentan-2,4-dion und 2,4,6-Trichlorphenylhydrazin als Ausgansstoffe, so läßt sich der Reaktionsablauf des erfindungsgemäßen Verfahrens (c) durch das folgende Formelschema darstellen:

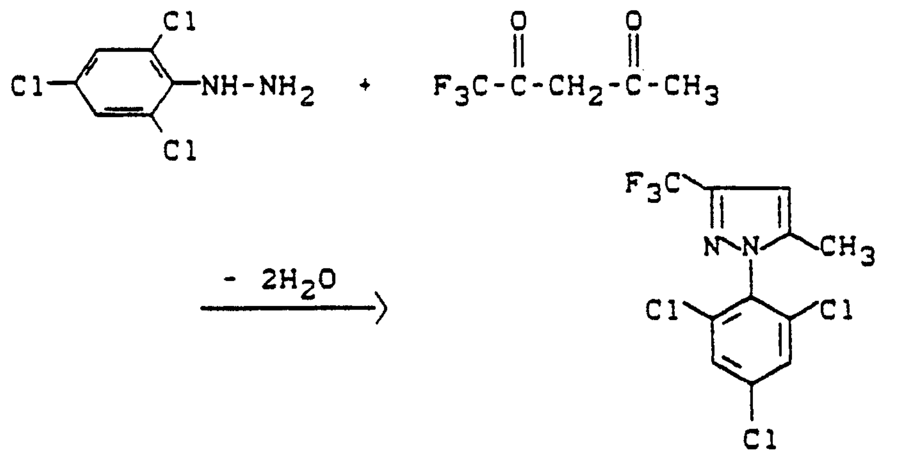


Verwendet man beispielsweise 3,5-Bis-(trifluormethyl)-1-(2,3,6-trichlor-4-trifluormethyl-phenyl)-pyrazol-4-yl-carbonsäure als Ausgangsverbindung, so läßt sich der Reaktionsablauf des erfindungsgemäßen Verfahren (d) durch das folgende Formelschema darstellen:

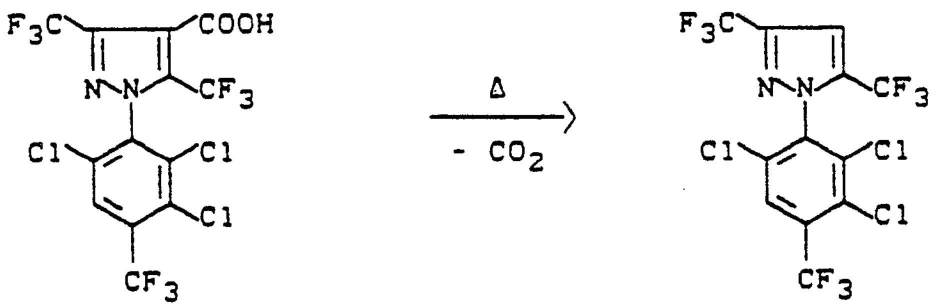


Die zur Durchführung des erfindungsgemäßen Verfahrens (a) als Ausgangsstoffe benötigten N-Arylhydrazid-halogenide sind durch die Formel (II) allgemein definiert. In dieser Formel (II) stehen $R^1$ und Ar vorzugsweise für diejenigen Reste, die bereits im Zusammenhang mit der Beschreibung der erfindungsgemäßen Stoffe der Formel (I) als bevorzugt für diese Substituenten genannt wurden. Hal steht vorzugsweise für Chlor oder Brom.

Die N-Arylhydrazid-halogenide der Formel (II) sind teilweise bekannt (vergl. z. B. Chem. Lett. 1982, 543: J. Heterocycl. Chem. 22, 565 [1985]; EP 1 019).

Man erhält sie beispielsweise, wenn man Arylhydrazine der Formel (VI),

$Ar\text{-}NH\text{-}NH_2$ (VI)

in welcher

Ar die oben angegebene Bedeutung hat,

mit Aldehyden der Formel (VIII),

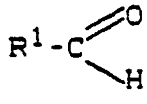

(VIII)

in welcher

$R^1$ die oben angegebene Bedeutung hat,

oder mit deren Hydraten oder Halbacetalen der Formel (IX),

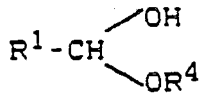 (IX)

in welcher
$R^1$ die oben angegebene Bedeutung hat und
$R^4$ für Wasserstoff oder Alkyl steht,
zunächst in einer ersten Stufe, gegebenenfalls in Gegenwart eines Verdünnungsmittels, wie beispielsweise Ethanol oder Toluol und gegebenenfalls in Gegenwart eines Reaktionshilfsmittels wie beispielsweise Schwefelsäure bei Temperaturen zwischen -30 °C und +150 °C umsetzt und die so erhältlichen Arylhydrazone der Formel (X),

$R^1$-CH=N-NH-Ar (X)

in welcher
$R^1$ und Ar die oben angegebene Bedeutung haben,
in einer 2. Stufe mit Halogenierungsmitteln wie beispielsweise N-Bromsuccinimid, N-Chlorsuccinimid oder Brom gegebenenfalls in Gegenwart eines Verdünnungsmittels wie beispielsweise Dimethylformamid oder Essigsäure bei Temperaturen zwischen -30 °C und +100 °C umsetzt.

Die Aldehyde der Formel (VIII), bzw. deren Hydrate oder Halbacetale der Formel (IX) sind allgemein bekannte Verbindungen der organischen Chemie (vergl. z. B. J. Am. chem. Soc., 76, 300 [1954]).

Die Arylhydrazone der Formel (X) sind teilweise bekannt (vgl. z. B. Bull. Chem. Soc. Japan 58, 1841 [1985]; J. Heterocycl. Chem. 22, 565 [1985] oder Chem. Lett. 1982, 543).

Die zur Durchführung des erfindungsgemäßen Verfahrens (a) weiterhin als Ausgangsstoffe benötigten Olefine sind durch die Formel (III) allgemein definiert. In dieser Formel (III) steht $R^2$ vorzugsweise für diejenigen Reste, die bereits im Zusammenhang mit der Beschreibung der erfindungsgmäßen Stoffe der Formel (I) als bevorzugt für diesen Substituenten genannt wurden. X steht vorzugsweise für jeweils geradkettiges oder verzweigtes Alkoxy, Alkylthio, Dialkylamino oder Trialkylsilyloxy mit jeweils 1 bis 4 Kohlenstoffatomen in den einzelnen Alkylteilen, für Morpholino oder für Halogen, insbesondere Chlor oder Brom.

Die Olefine der Formel (III) sind allgemein bekannte Verbindungen der organischen Chemie (vgl. z. B. Angew. Chem. 81, 374 [1969]; Synthesis 1977, 91; Tetrahedron 38, 1975 [1982]).

Die zur Durchführung des erfindungsgemäßen Verfahrens (b) als Ausgangsstoffe benötigten Pyrazoline sind durch die Formel (IV) allgemein definiert. In dieser Formel (IV) stehen $R^1$, $R^2$ und Ar vorzugsweise für diejenigen Reste, die bereits im Zusammenhang mit der Beschreibung der erfindungsgemäßen Stoffe der Formel (I) als bevorzugt für diese Substituenten genannt wurden. X steht vorzugsweise für jeweils geradkettiges oder verzweigtes Alkoxy, Alkylthio, Dialkylamino oder Trialkylsilyloxy mit jeweils 1 bis 4 Kohlenstoffatomen in den einzelnen Alkylteilen, für Morpholino oder für Halogen, insbesondere Chlor oder Brom.

Die Pyrazoline der Formel (IV) sind noch nicht bekannt. Man erhält sie in Analogie zu bekannten Verfahren (vgl. z. B. Chem. Lett. 1982, 543), wenn man N-Arylhydrazidhalogenide der Formel (II),

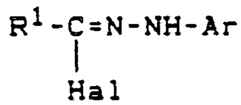 (II)

in welcher
$R^1$ und Ar die oben angegebene Bedeutung haben und
Hal für Halogen, insbesondere für Chlor oder Brom steht,
mit Olefinen der Formel (III),

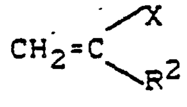 (III)

in welcher

$R^2$ un X die oben angegebene Bedeutung haben,
gegebenenfalls in Gegenwart eines Verdünnungsmittels wie beispielsweise Toluol und gegebenenfalls in Gegenwart eines Säurebindemittels wie beispielsweise Triethylamin bei Temperaturen zwischen 20 °C und 120 °C umsetzt.

Die zur Durchführung des erfindungsgemäßen Verfahrens (c) als Ausgangsstoffe benötigten 1,3-Diketone oder 1,3-Diketon-Derivate sind durch die Formeln (V) bzw. (Va) allgemein definiert. In diesen Formeln (V) bzw. (Va) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden. X in Formel (Va) steht vorzugsweise für jeweils geradkettiges oder verzweigtes Alkoxy, Alkylthio, Dialkylamino oder Trialkylsilyloxy mit jeweils 1 bis 4 Kohlenstoffatomen in den einzelnen Alkylteilen, für Morpholino oder für Halogen, insbesondere für Chlor oder Brom.

Die 1,3-Diketone der Formel (V) und die 1,3-Diketon-Derivate der Formel (Va) sind allgemein bekannte Verbindungen der organischen Chemie (vgl. z. B. Chem. Ber. 59, 1282 [1926]; Liebigs Ann. Chem. 452, 182 [1927]; J. org. Chemistry 21, 97 [1956]).

Die zur Durchführung des erfindungsgemäßen Verfahrens (c) und zur Synthese der Vorprodukte der Formel (II) weiterhin als Ausgangsstoffe benötigten Arylhydrazine sind durch die Formel (VI) allgemein definiert. In dieser Formel (VI) steht Ar vorzugsweise für diejenigen Reste, die bereits im Zusammenhang mit der Beschreibung der erfindungsgemäßen Stoffe der Formel (I) als bevorzugt für diesen Substituenten genannt wurden.

Die Arylhydrazine der Formel (VI) sind bekannt (vergl. z. B. EP 154 115, EP 187 285, EP 34 945) oder erhältlich nach allgemein bekannten Verfahren in Analogie zu bekannten Verbindungen (vergl. z. B. Houben-Weyl, "Methoden der organischen Chemie". Band X, 2, Thieme Verlag, Stuttgart 1967).

Die zur Durchführung des erfindungsgemäßen Verfahrens (d) als Ausgangsstoffe benötigten Pyrazolyl-4-carbonsäuren sind durch die Formel (VII) allgemein definiert. In dieser Formel (VII) stehen $R^1$, $R^2$ und Ar vorzugsweise für diejenigen Reste, die bereits im Zusammenhang mit der Beschriebung der erfindungsgemäßen Stoffe der Formel (I) als bevorzugt für diese Substituenten genannt wurden.

Die Pyrazolyl-4-carbonsäuren sind noch nicht bekannt. Sie sind jedoch Gegenstand einer eigenen parallelen Patentanmeldung und erhältlich in Analogie zu bekannten Verfahren (vgl. auch Bull. Chem. Soc. Japan 59, 2631 [1986]), beispielsweise, wenn man N-Aryl-hydrazidhalogenide der Formel (II),

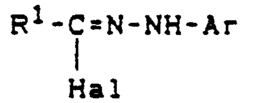 (II)

in welcher
Hal für Halogen steht und
$R^1$ und Ar die oben angegebene Bedeutung haben,
entweder

(α) mit Acylessigesterderivaten der Formel (XI),

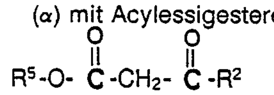 (XI)

in welcher
$R^2$ die oben angegebene Bedeutung hat und
$R^5$ für Alkyl steht,
oder

(β) mit β-Cyano-acrylsäureestern der Formel (XII),

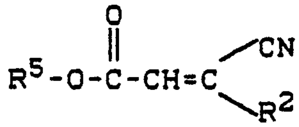 (XII)

in welcher
$R^2$ und $R^5$ die oben angegebene Bedeutung haben

oder

(γ) mit Alkincarbonestern der Formel (XIII),

$$R^5\text{-O-}\overset{\overset{\displaystyle O}{\|}}{C}\text{-C}\equiv\text{C-}R^2 \qquad \text{(XIII)}$$

in welcher

$R^5$ und $R^2$ die oben angegebene Bedeutung haben, jeweils gegebenenfalls in Gegenwart eines Verdünnungsmittels wie beispielsweise Ethanol oder Toluol und gegebenenfalls in Gegenwart eines Säurebindemittels, wie beispielsweise Natriumethylat oder Triethylamin bei Temperaturen zwischen 0 °C und 120 °C umsetzt, und die so erhältlichen 4-Alkoxycarbonylpyrazole der Formel (XIV),

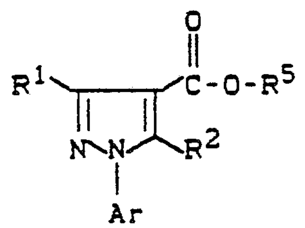

(XIV)

in welcher

$R^1$, $R^2$, $R^5$ und Ar die oben angegebenen Bedeutungen haben,

mit Säuren wie beispielsweise Schwefelsäure bei Temperaturen zwischen 20 °C und 100 °C in allgemein üblicher Art und Weise an der Estergruppe in 4-Position des Pyrazolringes verseift.

Die Acylessigesterderivate der Formel (XI), die β-Cyanoacrylsäureester der Foreml (XII) und die Alkincarbonester der Formel (XIII) sind allgemein bekannte Verbindungen der organischen Chemie (vgl. z. B. Organic Reactions 1, 266 [1947]; Synthesis 1984, 1 oder Houben-Weyl, "Methoden der organischen Chemie", Band V, 2a, S. 1, Thieme Verlag Stuttgart 1977).

Als Verdünnungsmittel zur Durchführung des erfindungsgemäßen Verfahrens (a) kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl-oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester.

Das erfindungsgemäße Verfahren (a) wird gegebenenfalls in Gegenwart eines geeigneten Säurebindemittels durchgeführt. Als solche kommen alle üblichen anorganischen oder organischen Basen in Frage. Vorzugsweise verwendet man tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen -30 °C und +150 °C, vorzugsweise bei Temperaturen zwischen 0 °C und 100 °C.

Zur Durchführung des erfindungsgemäßen Verfahrens (a) setzt man pro Mol an N-Arylhydrazidhalogenid der Formel (II) im allgemeinen 1.0 bis 20.0 Mol, vorzugsweise 1.0 bis 5.0 Mol an Olefin der Formel (III) und gegebenenfalls 1.0 bis 20.0 Mol, vorzugsweise 1.0 bis 2.0 Mol an Säurebindemittel ein.

Die Reacktionsdurchführung, Aufarbeitung und Isolierung der Reaktionsprodukte erfolgt nach allgemein üblichen Methoden. Es ist auch möglich, die bei der Durchführung des erfindungsgemäßen Verfahrens (a) als intermediäre Zwischenprodukte auftretenden Pyrazoline der Formel (IV) zu isolieren und in einer separaten Reaktionsstufe weiter umzusetzten (vgl. Verfahren (b) und die Herstellungsbeispiele).

Als Verdünnungsmittel zur Durchführung des erfindungsgemäßen Verfahrens (b) kommen ebenfalls inerte organische Lösungsmittel in Frage. Vorzugsweise verwendet man die bei Verfahren (a) genannten Lösungsmittel.

Das erfindungsgemäße Verfahren (b) wird in Gegenwart einer geeigneten Katalysatorsäure durchgeführt. Als solche kommen alle üblichen anorganischen oder organischen Säuren in Frage, Vorzugsweise verwendet man Chlorwasserstoffsäure, Schwefelsäure oder p-Toluolsulfonsäure.

Die Reaktionstemperaturen können bei der Durchführung des erfindungsgemäßen Verfahrens (b) in einem größeren Bereich variiert werden. Im allgemeinen arbeitet man bei Temperaturen zwischen o °C und 150 °C, vorzugsweise bei Temperaturen zwischen 20 °C und 130 °C.

Zur Durchführung des erfindungsgemäßen Verfahrens (b) setzt man pro Mol an Pyrazolin der Formel

(IV) im allgemeinen 0.001 bis 10.0 Mol, vorzugsweise 0.1 bis 1.0 Mol an Katalysatorsäure ein. Die Reaktionsdurchführung, Aufarbeitung und Isolierung der Reaktionsprodukte der Formel (I) erfolgt nach allgemein üblichen Verfahren. In einer besonderen Ausführungsform des erfindungsgemäßen Verfahrens (b) ist es auch möglich, die als Ausgangsverbindungen benötigten Pyrazoline der Formel (IV) direkt im Reaktionsgefäß herzustellen [durch Umsetzung von Verbindungen der Formel (II) mit Verbindungen der Formel (III) wie vorstehend beschrieben] und aus dem Reaktionsgemisch heraus in einem "Eintopfverfahren" weiter umzusetzen (vgl. auch die Herstellungsbeispiele).

Als Verdünnungsmittel zur Durchführung des erfindungsgemäßen Verfahrens (c) kommen inerte organische Lösungsmittel in Frage, Vorzugsweise verwendet man die bei Verfahren (a) genannten Verdünnungsmittel oder polare Lösungsmittel wie Essigsäure, Methanol, Ethanol, Propanol oder Butanol.

Die Reaktionstemperaturen können bei der Durchführung des erfindungsgemäßen Verfahrens (c) in einem größeren Bereich variiert werden. Im allgemeinen arbeitet man bei Temperaturen zwischen -50 °C und +200 °C, vorzugsweise bei Temperaturen zwischen 0 °C und 150 °C.

Zur Durchführung des erfindungsgemäßen Verfahrens (c) setzt man pro Mol an Arylhydrazin der Formel (VI) im allgemeinen 1.0 bis 10.0 Mol, vorzugsweise 1.0 bis 2.0 Mol an 1,3-Diketon der Formel (V) bzw. eines 1,3-Diketon-Derivates der Formel (Va) ein.

Die Reaktionsdurchführung, Aufarbeitung und Isolierung der Reaktionsprodukte erfolgt in Analogie zu allgemein üblichen Methoden.

Als Verdünnungsmittel zur Durchführung des erfindungsgemäßen Verfahrens (d) kommen inerte organische Lösungsmittel in Frage. Vorzugsweise verwendet man höhersiedende alicyclische oder aliphatische Kohlenwasserstoffe oder Aromaten wie beispielsweise Dekalin oder Chinolin.

Das erfindungsgemäße Verfahren (d) wird gegebenenfalls in Gegenwart eines geeigneten Reaktionshilfsmittels durchgefüht. Als solche kommen alle üblicherweise für Decarboxylierungsreaktionen verwendbaren Katalysatoren in Frage. Vorzugsweise verwendet man Metallkatalysatoren wie beispielsweise Kupfer.

Die Reaktionstemperaturen können bei der Durchführung des erfindungsgemäßen Verfahrens (d) in einem größeren Bereich variiert werden. Im allgemeinen arbeitet man bei Temperaturen zwischen 50 °C und 250 °C, vorzugsweise bei Temperaturen zwischen 100 °C und 200 °C.

Zur Duchführung des erfindungsgemäßen Verfahrens (d) setzt man im allgemeinen pro Mol an Pyrazolyl-4-carbonsäure der Formel (VII) 0.001 bis 10.0 Mol, vorzugsweise 0.01 bis 2.0 Mol an Katalysator ein.

Die Reaktionsdurchführung, Aufarbeitung und Isolierung der Reaktionsprodukte erfolgt in Analogie zu allgemein üblichen Methoden (vgl. z. B. C. Ferri, "Reaktionen der organischen Synthese" S. 335 - 340, Thieme Verlag, Stuttgart 1978).

Die Wirkstoffe eignen sich zur Bekämpfung von tierischen Schädlingen, vorzugsweise Arthropoden und insbesondere Insekten, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum,

Hyalopterus arundinis, Macrosiphum avenae, Myzus spp., Phorodon humuli, Rhopalosiphum padi, Empoasca spp., Euscelisbilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp..

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solsti tialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Os cinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Dabei lassen sich die erfindungsgemäßen Wirkstoffe mit besonders gutem Erfolg zur Bekämpfung von Blatt-und Bodeninsekten, wie beispielsweise gegen die Larven der Meerrettichblattkäfer (Phaedon cochleariae) oder gegen die Maden der Zwiebelfliege (Phorbia antiqua) einsetzen. Darüberhinaus eignen sich die erfindungsgemäßen Wirkstoffe auch sehr gut zur Bekämpfung von Hygiene-und Vorratsschädlingen, wie beispielsweise die gemeine Stubenfliege (Musca domestica) oder die deutsche Hausschabe (Blattella germanica). Daneben eignen sich die erfindungsgemäßen Wirkstoffe auch zur Bekämpfung von pilzlichen Schädlingen und lassen sich beispielsweise zur Bekämpfung des echten Getreidemehltaus (Erysiphe graminis) einsetzen.
Dabei zeigen die erfindungsgemäßen Wirkstoffe neben guten protektiven auch systemische Eigenschaften.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmittlen oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse

Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder - schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise. 00000000

Herstellungsbeispiele

Beispiel 1

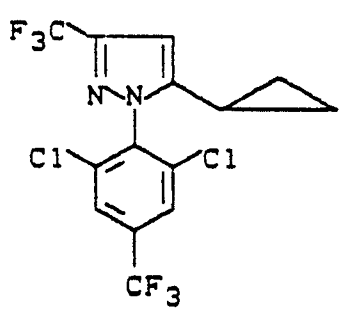

(Verfahren a)

Zu einer Lösung von 20,2 g (0.05 Mol) N-(2,6-Dichlor-4-trifluoromethylphenyl)-trifluoracethydrazid-bromid in 30 ml Toluol tropft man bei 90 ° C unter Rühren ein Gemisch von 7,65 g (0.05 Mol) 1-Cyclopropyl-1-morpholinoethen vgl. Tetrahedron 31, 2427 - 2429 [1975]) und 5,05 g (0.05 Mol) Triethylamin und erhitzt anschließend noch 2 Stunden auf 90 °C. Nach Abkühlen wird vom Triethylammoniumbromid

abfiltriert und eingeengt.

Durch säulenchromatographische Auftrennung und Kugelrohrdestillation bei 150 °C (0.07 Torr) erhält man 6,71 g (34,5 % der Theorie) 5-Cyclopropyl-3-trifluormethyl-1-(2,6-dichlor-4-trifluormethylphenyl)-pyrazol vom Brechungsindex $n_D^{20}$ : 1.487.

Beispiel 2

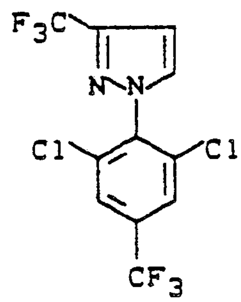

(Verfahren b)

Zu 68,4 g (0.16 Mol) 5-Butoxy-3-trifluormethyl-1-(2,6-dichlor-4-trifluormethylphenyl)-pyrazolin in 60 ml Acetonitril fügt man eine Spatelspitze p-Toluolsulfonsäure und kocht 12 Stunden unter Rückfluß. Nach Abkühlen auf Raumtemperatur neutralisiert man mit Natriumbicarbonat und engt ein. Der Rückstand wird in Petrolether aufgenommen, filtriert und wieder eingeengt.

Durch Kugelrohrdestillation bei 120 °C (0.08 Torr) erhält man 49,7 g (89 % der Theorie) 3-Trifluormethyl-1-(2,6-dichlor-4-trifluormethylphenyl)-pyrazol als Öl, vom Brechungsindex $n_D^{20}$ : 1.476.

Beispiel 3

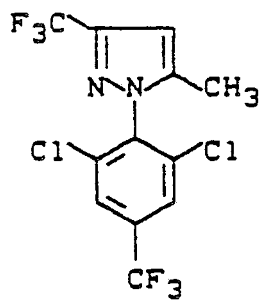

(Verfahren a/b "Eintopfverfahren")

Man tropft ein Gemisch von 7,2 g (0.1 Mol) 2-Propenylmethylether, 10,1 g (0.1 Mol) Triethylamin und 10 ml Toluol bei 90 °C unter Rühren zu einer Lösung von 20,2 g (0.05 Mol) N-(2,6-Dichlor-4-trifluormethylphenyl)-trifluoracethydrazid-bromid in 30 ml Toluol. Nach beendeter Zugabe erhitzt man 4,5 Stunden auf 90 °C, kühlt ab auf Raumtemperatur, saugt ausgefallenes Triethylammoniumbromid ab und engt ein. Der Rückstand wird in Ether aufgenommen und mit einer Spatelspitze p-Toluolsulfonsäure versetzt. Nach 5 Stunden bei Raumtemperatur neutralisiert man mit Natriumhydrogencarbonat und engt ein.

Durch Säulenchromatographie über Kieselgel erhält man 10,74 g (59,2 % der Theorie) 5-Methyl-3-trifluormethyl-1-(2,6-dichlor-4-trifluormethylphenyl)-pyrazol vom Schmelzpunkt 77 °C - 79 °C.

In entsprechender Weise und gemäß den allgemeinen Angaben zur Herstellung erhält man die folgenden substituierten 1-Arylpyrazole der allgemeinen Formel (I):

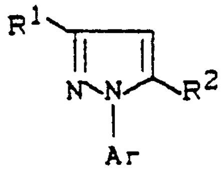

(I)

| Bsp.-Nr. | $R^1$ | $R^2$ | Ar | physikalische Eigenschaften |
|---|---|---|---|---|
| 4 | $CF_3$ | H | 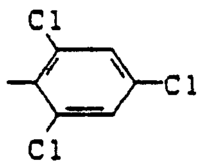 | Kp. 150 °C/0.1 mbar<br>Fp. 66 °C - 67,5 °C |
| 5 | $CF_3$ | H | 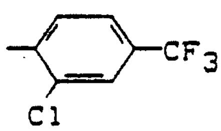 | Kp. 125 °C/0.11 mbar |
| 6 | $CF_3$ | H | 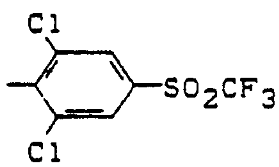 | Kp. 190 °C/0.1 mbar<br>Fp. 87 °C - 89 °C |
| 7 | $CF_3$ | H | 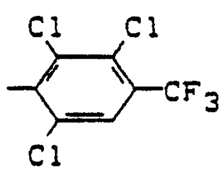 | Kp. 160 °C/0.07 mbar |

<u>Herstellung der Ausgangsverbindungen</u>

Beispiel IV-1

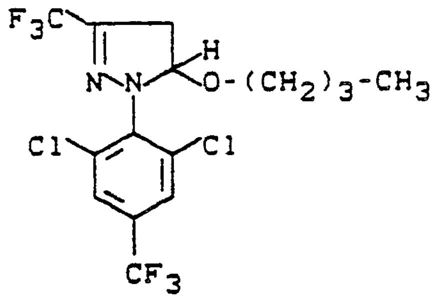

Zu einer Lösung von 4.04 g (0.01 Mol) N-(2,6-Dichlor-4-trifluormethylphenyl)-trifluoracethydrazid-bromid in 2,5 ml Toluol tropft man bei 90 °C langsam ein Gemisch von 2,0 g (0.02 Mol) n-Butylvinylether, 2,02 g (0.02 Mol) Triethylamin und 2 ml Toluol. Nach 3 Stunden Rühren bei 90 °C läßt man das Reaktionsgemisch auf Raumtemperatur kommen und filtriert vom ausgefallenen Triethylammoniumbromid ab.

Einengen und kugelrohrdestillation bei 160 °C (0.04 Torr) ergibt 3,50 g (83 % der Theorie) 5-Butoxy-3-trifluormethyl-1-(2,6-dichlor-4-trifluormethylphenyl)-pyrazolin als Öl, vom Brechungsindex $n_D^{20}$ : 1.476.

In entsprechender Weise und gemäß den allgemeinen Angaben zur Herstellung erhält man die folgenden Pyrazoline der allgemeinen Formel (IV):

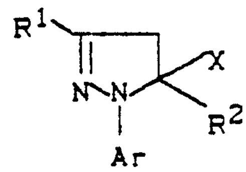

(IV)

| Bsp.-Nr. | $R^1$ | $R^2$ | X | Ar | Siedepunkt /°C(bei mbar) |
|---|---|---|---|---|---|
| IV-2 | $CF_3$ | H | $-O-(CH_2)_3-CH_3$ | 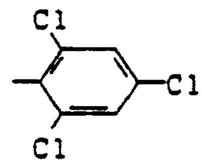 | 160 (0.08) |
| IV-3 | $CF_3$ | H | $-O-(CH_2)_3-CH_3$ | 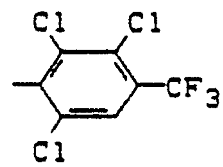 | 170 (0.05) |
| IV-4 | $CF_3$ | H | $-O-(CH_2)_3-CH_3$ | 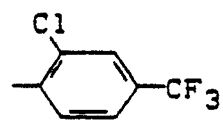 | 155 (0.07) |
| IV-5 | $CF_3$ | H | $-O-(CH_2)_3-CH_3$ | 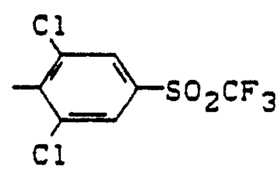 | 205 (0.06) |

Beispiel II-1

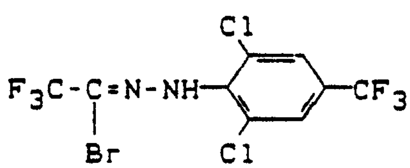

Zu einer Lösung von 8,12 g (0.025 Mol) Trifluoracetaldehyd-N-(2,6-dichlor-4-trifluormethylphenyl)-hydrazon in 7,5 ml Dimethylformamid gibt man bei Raumtemperatur portionsweise 4,45 g (0.025 Mol) N-Bromsuccinimid, wobei eine exotherme Reaktion auftritt. Man rührt 3 Stunden bei Raumtemperatur, destilliert das Dimethylformamid ab und versetzt den Rückstand mit 20 ml Petrolether. Nach Absaugen des ausgefallenen Succinimids wird das Filtrat eingeengt und einer Kugelrohrdestillation unterworfen.

Man erhält 9,26 g (91,7 % der Theorie) N-(2,6-Dichlor-4-trifluormethylphenyl)-trifluoracethydrazid-bromid vom Siedepunkt 110 °C bei 0,06 mbar und vom Brechungsindex $n_D^{20}$ 1,510.

Beispiel II-2

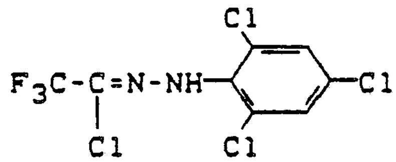

3,2 g (0.011 Mol) Trifluoracetaldehyd N-(2,4,6-Trichlorphenyl)-hydrazon und 1,5 g (0.011 Mol) N-Chlorsuccinimid in 3,3 ml Dimethylformamid werden 3 Stunden bei Raumtemperatur gerührt. Nach Einengen versetzt man mit Petrolether, filtriert und engt wieder ein.

Kugelrohrdestillation des Rückstands bei 150 °C (0,5 mbar) liefert 3,28 g (89,6 % der Theorie) N-(2,4,6-Trichlorphenyl)-trifluoracethydrazid-chlorid vom Brechungsindex $n_D^{20}$ 1,549.

In entsprechender Weise und gemäß den allgemeinen Angaben zur Herstellung erhält man die folgenden N-Arylhydrazid-halogenide der allgemeinen Formel (II):

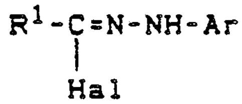 (II)

| Bsp.-Nr. | $R^1$ | Hal | Ar | physikalische Eigenschaften |
|---|---|---|---|---|
| II-3 | $CF_3$ | Br | 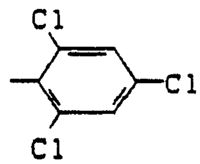 | Kp. 125 °C/ 0,08 mbar |
| II-4 | $CF_3$ | Cl | 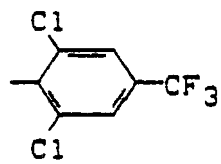 | Kp. 130 °C/ 0,5 mbar |
| II-5 | $CF_3$ | Br | 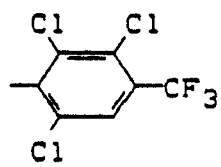 | Fp. 62 - 63°C |
| II-6 | $CF_3$ | Br | 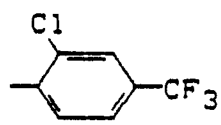 | Kp. 95 °C/ 0,05 mbar |
| II-7 | $CF_3$ | Cl | 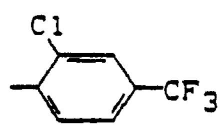 | Kp. 90 °C/ 0,13 mbar |
| II-8 | $CF_3$ | Br | 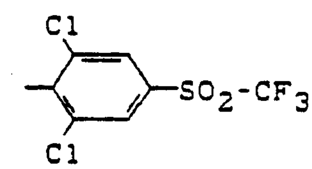 | Fp. 48 - 50°C |

Beispiel X-1

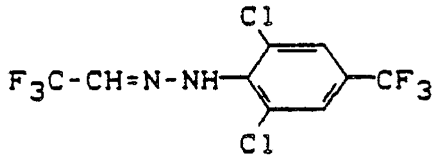

Ein Gemisch von 100 g (0.69 Mol) Trifluoracetyaldehydethylhalbacetal und 169,1 g (0.69 Mol) 2,6-Dichlor-4-methylphenylhydrazin wird 6 Stunden bei 100 °C erhitzt. Nach Abdestillieren der flüchtigen Anteile im Vakuum wird der Rückstand aus Petrolether umkristallisiert.

Man isoliert 200 g (89 % der Theorie) Trifluoracetaldehyd-N-(2,6-dichlor-4-trifluormethylphenyl)-hydrazon vom Schmelzpunkt 45 °C bis 46 °C.

In entsprechender Weise und gemäß den allgemeinen Angaben zur Herstellung erhält man die folgenden Aldehyd-N-arylhydrazone der allgemeinen Formel (X):

$R^1$-CH = N-NH-Ar (X)

| Bsp.-Nr. | $R^1$ | Ar | Schmelzpunkt /° C |
|---|---|---|---|
| X-2 | $CF_3$ | 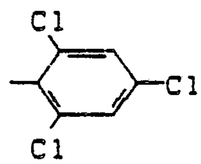 | 43 - 45 |
| X-3 | $CF_3$ | 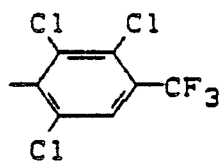 | 50 - 52 |
| X-4 | $CF_3$ | 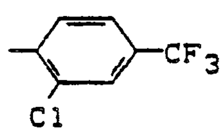 | 63 - 65 |
| X-5 | $CF_3$ | 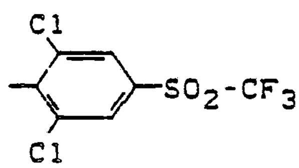 | 124 - 126 |

Anwendungsbeispiele

In den folgenden Anwendungsbeispielen wurden die nachstehend aufgeführten Verbindungen als Vergleichssubstanzen eingesetzt:

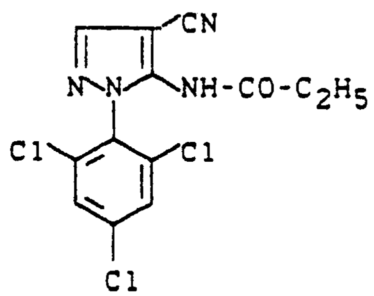

(A)

4-Cyano-5-propionamido-1-(2,4,6-trichlorphenyl)-pyrazol

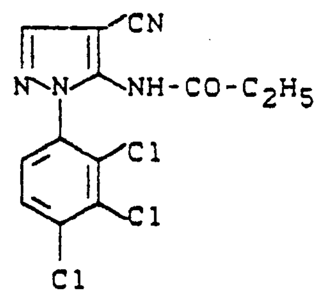

(B)

4-Cyano-5-propionamido-1-(2,3,4-trichlorphenyl)-pyrazol

(beide bekannt aus DE-OS 32 26 513).

Beispiel A

Phaedon-Larven-Test
Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigen z. B. die folgenden Verbindungen der Herstellungsbeispiele überlegene Wirksamkeit gegenüber dem Stand der Technik: 2 und 6.

Beispiel B

Grenzkonzentrations-Test / Bodeninsekten
Testinsekt: Phorbia antiqua-Maden im Boden
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik: 2 und 6.

Beispiel C

$LT_{100}$-Test für Dipteren
Testtiere: Musca domestica
Zahl der Testtiere: 20
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z.B. die folgenden Verbindungen der Herstellungsbeispiele überlegene Wirkung gegenüber dem Stand der Technik: 2 und 6.

Beispiel D

LD100-Test

Testtiere: Blattella germanica Zahl der Testtiere: etwa 20 Lösungsmittel: Aceton
2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden; 0 % bedeutet, daß keine Testtiere abgetötet wurden.

Bei diesem Test zeigen z. B. die folgenden Verbindungen der Herstellungsbeispiele überlegene Wirkung gegenüber dem Stand der Technik: 2 und 6.

**Ansprüche**

1. Substituierte 1-Arylpyrazole der allgemeinen Formel (I)

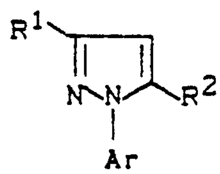

(I) .

in welcher
$R^1$ für Halogenalkyl steht,
$R^2$ für Wasserstoff, Alkyl, Cycloalkyl oder Halogenalkyl steht und
Ar für substituiertes Phenyl mit Ausnahme des 4-Nitrophenylrestes, des 2,4-Dinitrophenylrestes und mit Ausnahme von 2,4-Dihalogen-5-alkoxysubstituierten Phenylresten steht.

2. Substituierte 1-Arylpyrazole gemäß der Formel (I) von Anspruch 1, dadurch gekennzeichnet, daß
$R^1$ für geradkettiges oder verzweigtes Halogenalkyl mit 1 bis 4 Kohlenstoffatomen und 1 bis 9 gleichen oder verschiedenen Halogenatomen steht,
$R^2$ für Wasserstoff, für jeweils geradkettiges oder verzweigtes Alkyl oder Halogenalkyl mit jeweils 1 bis 4 Kohlenstoffatomen und im Fall des Halogenalkyl mit 1 bis 9 gleichen oder verschiedenen Halogenatomen oder für Cycloalkyl mit 3 bis 7 Kohlenstoffatomen steht und
Ar für einfach oder mehrfach gleich oder verschieden substituiertes Phenyl mit Ausnahme des 4-Nitrophenylrestes, des 2,4-Dinitrophenylresstes und mit Ausnahme von 2,4-Dihalogen-5-alkoxy-substituierten Phenylresten steht, wobei als Substituenten in Frage kommen: Halogen, Cyano, Nitro sowie jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl oder Alkoxycarbonyl mit jeweils 1 bis 4 Kohlenstoffatomen in den einzelnen Alkylteilen und gegebenenfalls 1 bis 9 gleichen oder verschiedenen Halogenatomen.

3. Substituierte 1-Arylpyrazole gemäß der Formel (I) von Anspruch 1, dadurch gekennzeichnet, daß
$R^1$ für Trifluormethyl, Dichlorfluormethyl, Difluorchlormethyl, Difluormethyl, Trifluorethyl, Trifluorchlorethyl, Trifluordichlorethyl, Difluordichlorethyl, Tetrafluorethyl, Pentafluorethyl, Heptafluorpropyl oder Nonafluorbutyl steht,
$R^2$ für Wasserstoff, Methyl, Ethyl, n-oder i-Propyl, n-, i-, s-oder t-Butyl, Trifluormethyl, Dichlorfluormethyl, Difluorchlormethyl, Difluormethyl, Trifluorethyl, Tetrafluorethyl, Pentafluorethyl, Trifluorchlorethyl, Trifluordichlorethyl, Difluordichlorethyl, Heptafluorpopyl, Nonafluorbutyl, für Cyclopropyl, Cyclobutyl, Cyclopentyl oder Cyclohexyl steht und
Ar für ein-bis fünffach, gleich oder verschieden substituiertes Phenyl mit Ausnahme des 4-Nitrophenylrestes, des 2,4-Dinitrophenylrestes und mit Ausnahme von 2,4-Dihalogen-5-alkoxy-substituierten Phenylresten

steht, wobei als Substituenten in Frage kommen: Fluor, Chlor, Brom, Iod, Cyano, Nitro, Methyl, Ethyl, n-oder i-Propyl, n-, i-, s-oder t-Butyl, Methoxy, Ethoxy, Methylthio, methylsulfinyl, methylsulfonyl, Methoxycarbonyl, Ethoxycarbonyl, Trifluormethyl, Dichlorfluormethyl, Difluorchlormethyl, Difluormethyl, Pentafluorethyl, Tetrafluorethyl, Trifluorchlorethyl, Trifluorethyl, Difluorchlorethyl, Trifluordichlorethyl oder ein Rest -Y-$R^3$, wobei Y für Sauerstoff, Schwefel, Sulfinyl oder Sulfonyl steht und $R^3$ für Trifluormethyl, Dichlorfluormethyl, Difluorchlormethyl, Difluormethyl, Pentafluorethyl, Tetrafluorethyl, Trifluorchlorethyl, Trifluorethyl, Difluorchlorethyl oder Trifluordichlorethyl steht.

4. Verfahren zur Herstellung von substituierten 1-Arylpyrazolen der allgemeinen Formel (I)

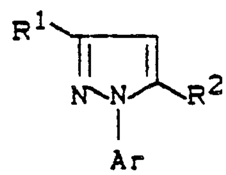

(I)

in welcher

$R^1$ für Halogenalkyl steht,

$R^2$ für Wasserstoff, Alkyl, Cycloalkyl oder Halogenalkyl steht und

Ar für substituiertes Phenyl mit Ausnahme des 4-Nitrophenylrestes, des 2,4-Dinitrophenylrestes und mit Ausnahme von 2,4-Dihalogen-5-alkoxy-substituierten Phenylresten steht,

dadurch gekennzeichnet, daß man

(a) N-Arylhydrazid-halogenide der Formel (II),

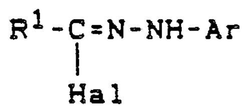

(II)

in welcher

$R^1$ und Ar die oben angegebene Bedeutung haben und

Hal für Halogen steht,

mit Olefinen der Formel (III),

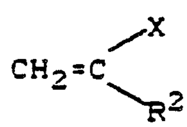

(III)

in welcher

$R^2$ die oben angegebene Bedeutung hat und

X für Alkoxy, Alkylthio, Dialkylamino, Morpholino, Halogen oder Trialkylsilyloxy steht,

gegebenenfalls in Gegenwart eines Verdünnungsmittels und gegebenenfalls in Gegenwart eines Säurebindemittels umsetzt oder daß man

(b) Pyrazoline der Formel (IV),

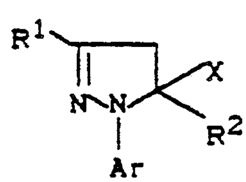

(IV)

in welcher

$R^1$, $R^2$ und Ar die oben angegebene Bedeutung haben und
X für Alkoxy, Alkylthio, Dialkylamino, Morpholino, Halogen oder Trialkylsilyloxy steht,
mit Säuren gegebenenfalls in Gegenwart eines Verdünnungsmittels umsetzt oder daß man

(c) 1,3-Diketone der Formel (V),

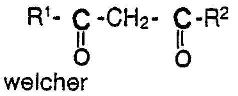 (V)

in welcher
$R^1$ und $R^2$ die oben angegebene Bedeutung haben
oder Derivate dieser 1,3-Diketone der Formel (Va),

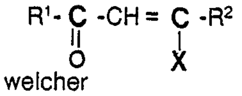 (Va)

in welcher
$R^1$ und $R^2$ die oben angegebene Bedeutung haben und
X für Alkoxy, Alkylthio, Dialkylamino, Morpholino, Halogen oder Trialkylsilyloxy steht,
mit Arylhydrazinen der Formel (VI),

$Ar-NH-NH_2$ (VI)

in welcher
Ar die oben angegebene Bedeutung hat,
gegebenenfalls in Gegenwart eines Verdünnungsmittels umsetzt oder daß man

d) Pyrazolyl-4-carbonsäuren der Formel (VII),

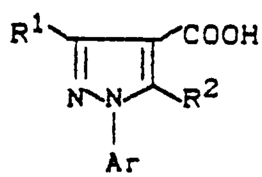 (VII)

in welcher
$R^1$, $R^2$ und Ar die oben angegebene Bedeutung haben,
gegebenenfalls in Gegenwart eines Verdünnungsmittels und gegebenenfalls in Gegenwart eines Reaktionshilfsmittels decarboxyliert.

5. Schädlingsbekämpfungsmittel, gekennzeichnet durch einen Gehalt an mindestens einem substituierten 1-Arylpyrazol der Formel (I).

6. Verfahren zur Bekämpfung von tierischen Schädlingen, dadurch gekennzeichnet, daß man substituierte 1-Arylpyrazole der Formel (I) auf tierische Schädlinge und/oder ihren Lebensraum einwirken läßt.

7. Verwendung von substituierten 1-Arylpyrazolen der Formel (I) zur Bekämpfung von tierischen Schädlingen.

8. Verfahren zur Herstellung von Mitteln gegen tierische Schädlinge, dadurch gekennzeichnet, daß man substituierte 1-Arylpyrazole der Formel (I) mit Streckmitteln und/oder oberflächenaktiven Mitteln vermischt.